(19) Europäisches Patentamt
European Patent Office
Office européen des brevets

(11) Publication number: **0 213 893 B1**

(12) **EUROPEAN PATENT SPECIFICATION**

(45) Date of publication of patent specification: **10.11.93**

(51) Int. Cl.⁵: **C08G 59/68**, C07D 309/34

(21) Application number: **86306476.2**

(22) Date of filing: **21.08.86**

# (54) Photopolymerisable compositions containing substituted pyrylium salts as photoinitiators; and novel pyrylium salts for use therein.

(30) Priority: **02.09.85 IE 2158/85**

(43) Date of publication of application:
**11.03.87 Bulletin 87/11**

(45) Publication of the grant of the patent:
**10.11.93 Bulletin 93/45**

(84) Designated Contracting States:
**DE FR GB**

(56) References cited:
**EP-A- 0 176 777**
**US-A- 4 139 655**
**US-A- 4 451 659**

**POLYMER PREPRINTS, March 1982; A. LEDWITH, pp. 323-324&NUM;**

**PATENT ABSTRACTS OF JAPAN, vol. 8, no. 266 (C-255)[1703], 06 December 1984&NUM;**

**CHEMICAL ABSTRACTS, vol. 83, no. 2, 14 July 1975, Columbus, OH (US); V.P. KARMAZIN et al., p. 517, no. 17984k&NUM;**

**CHEMICAL ABSTRACTS, vol. 77, no. 16, 16 October 1972, Columbus, OH (US); A. MISTR et al., p. 424, no. 107686v&NUM;**

**THE JOURNAL OF ORGANIC CHEMISTRY, vol. 33, no. 3, March 1968; J.R. VANALLEN et al., pp. 1102-1105&NUM;**

**POLYMER SCIENCE DICTIONARY, Elsevier Science Publishers, 1989; M.S.M. ALGER, p. 151&NUM;**

(73) Proprietor: **Loctite (Ireland) Limited**
**Whitestown Industrial Estate**
**Dublin 24(IE)**

(72) Inventor: **Woods, John G.**
**138 Stillorgan Wood**
**Upper Kilmacud Road, Cy. Dublin(IE)**
Inventor: **Rooney, John M.**
**64 Water Street**
**South Glastonbury, CT 06073(US)**

(74) Representative: **Marchant, James Ian et al**
**Elkington and Fife**
**Prospect House**
**8 Pembroke Road**
**Sevenoaks, Kent TN13 1XR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to photopolymerisable compositions capable of polymerisation by an acid-catalysed mechanism under the influence of ultra-violet and/or visible light.

The use of Lewis Acid catalysts in cationically polymerisable compositions is well known, as discussed in US-A-4 058 400 Crivello, for example. The development of photocurable cationic systems is of great interest because they are not sensitive to oxygen and can be made up in one-package formulations which do not require mixing of ingredients or heat at the point of use. US-A-4 139 655 Tsao describes photo-curable compositions comprising an epoxy resin and a photo-decomposable thiopyrylium salt of the formula

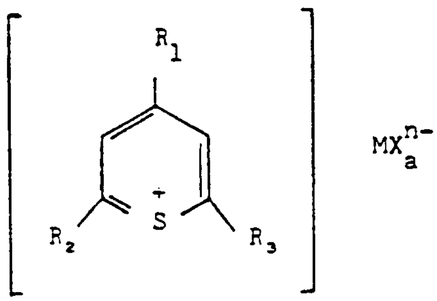

where $R_1$, $R_2$ and $R_3$ may be hydrogen, alkyl containing 1 to 10 carbon atoms, cycloalkyl containing 5 to 12 carbon atoms, aryl containing 6 to 12 carbon atoms and substituted derivatives thereof, M is a metal or metalloid, X is a halogen radical, a is a whole number from 1 to 10 and n is a whole number from 1 to 3. Typical $MXa^{n-}$ anions include $BF_4^-$, $PF_6^-$ $AsF_6^-$, $SbF_6^-$ and the like. Among the thiopyrylium salts described therein as catalysts are 2,6-diphenyl-4-p-methoxyphenyl-thiopyrylium tetrafluoroborate, 2,6-diphenyl-4-p-methoxyphenyl-thiopyrylium hexafluorophosphate, 2,4-6-tri-p-methoxyphenyl-thiopyrylium tetrafluorborate and 2,4,6-tri-p-methoxyphenyl-thiopyrylium hexafluorophosphate.

US-A-4 405 765 Demmler et al describes a curable resin composed of an epoxy resin, and a pyrylium salt as a catalyst, with or without a metal chelate or metal salt as a co-catalyst. The pyrylium salt is of the formula

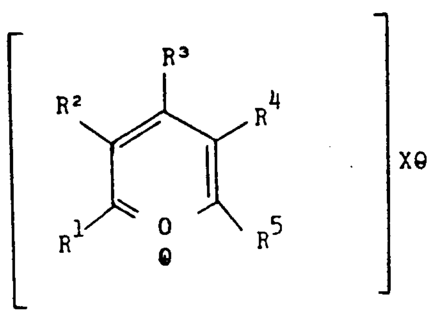

where $R^1$ and $R^5$ are identical and are each alkyl or aralkyl, each of 1 to 8 carbon atoms, and $R^2$, $R^3$ and $R^4$ are identical or different and are each hydrogen or alkyl, alkaryl or cycloalkyl, each of not more than 8 carbon atoms or an unsubstituted or alkyl substituted phenyl , naphthyl, indenyl, indanyl or fluorenyl group or a heterocyclic substituent containing one or two heteroatoms. $X^-$ is a non-nucleophilic, weakly basic anion such as $AsF_6^-$, $SbF_6^-$, $BF_4^-$, $PF_6^-$, $CF_3SO_3^-$ or $ClO_4^-$, preferably $AsF_6^-$.

US-A-4 474 868 Yamaoko et al describes a photo-polymerisation initiator composition comprising an organic peroxide and a pyrylium salt, thiapyrylium salt or selenopyrylium salt of the formula

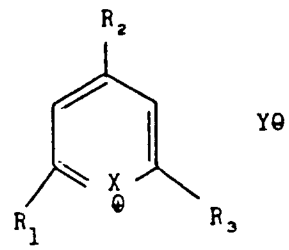

wherein $R_1$, $R_2$ and $R_3$ each represents a hydrogen atom, an alkyl group, a haloalkyl group, an ethylenyl group, a styryl group, an alkoxy group, a phenyl group, a naphthyl group, an alkylphenyl group, an alkoxyphenyl group, a hydroxyphenyl group, a halophenyl group, a nitrophenyl group, an aminophenyl group, a nitro group, an amino group or a hydroxyl group, X represents an oxygen atom, a sulfur atom or a selenium atom, and Y represents an anion functional group.

Among the salts of formula 1 are 4-(4-butoxyphenyl)-2,6-diphenylpyrylium perchlorate; 2,6-bis (4-ethylphenyl)-4-(4-methoxyphenyl)-pyrylium fluoroborate; 4-(4-butoxyphenyl)-2,6-bis (4-methoxyphenyl)-thiapyrylium fluoroborate; 2,6-bis (4-ethylphenyl)-4-(4-methoxyphenyl)-pyrylium fluoroborate; 4-(4-butoxyphenyl)-2,6-diphenyl thiapyrylium perchlorate, fluoroantimonate, fluorophosphate or fluoroborate; 2,4,6-tri (4-methoxyphenyl)thiapyrylium perchlorate; and 2,6-bis (4-methoxyphenyl)-4-phenyl thiapyrylium fluoroborate.

In a publication entitled "Photochemically initiated epoxide polymerisations" in Polymer Preprints, March 1982, 323-324, A. Ledwith has reported that triarylpyrylium salts of the formula

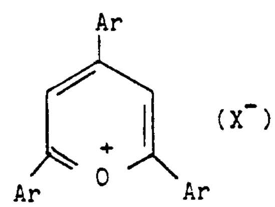

Ar = phenyl, p-tolyl

are extremely effective photoinitiators for cyclohexene oxide polymerisation if the anion is $SbF_6-$, whereas poor results were obtained for $BF_4-$ salts and $PF_6-$ salts.

The present inventors have carried out further work on the use of phenyl substituted pyrylium salts containing $SbF_6-$ anion as cationic photoinitiators. However they have found that the usefulness of these initiators is limited since the stability in the dark of compositions containing these initiators is poor.

The present inventors have found surprisingly that if a phenyl substituent bearing an electron donor group is present on the pyrylium salt, then the long term dark stability of a photopolymerisable composition is greatly increased, making it useful for commercial applications.

The invention therefore provides a photopolymerisable composition comprising

a) at least one-epoxide compound or epoxy resin, and

b) a pyrylium salt having at least one phenyl substituent bearing an electron donor group in the para- and/or ortho-position on the phenyl ring, and a hexafluoroantimonate or hexafluoroarsenate counterion.

The electron donor group may be selected from the known range of such groups which can be substituted on a phenyl group, for example:-

hydrocarbyloxy (-OR)
hydroxy (-OH)
amide ($-NHCOCH_3$)
phenoxy (-O Ar)
thiohydrocarbyl (-SR)
thioaryl (-SAr)
phenyl ester (-OCOR)

wherein R represents hydrocarbyl and Ar represents aryl.

The preferred electron donor group is hydrocarbyloxy, more particularly alkoxy or alkenoxy.

The pyrylium salt may suitably be added in an amount from 0.01-20%, preferably 0.1-10%, and more particularly 0.5-3%, by weight of the weight of the composition.

The pyrylium salt may be synthesised by known methods as described for example in US-A-4,139,655 Tsao. This patent describes the preparation of 2,4,6-triphenylpyrylium salts by a coupling and cyclisation reaction of acetophenone and benzaldehyde. Pyrylium salts containing phenyl substituents bearing hydrocarbyloxy electron donor groups can be prepared by this procedure using the corresponding hydrocarbyloxy substituted acetophenone and benzaldehyde derivatives.
Examples of the syntheses of such derivatives are given below where the hydrocarbyloxy group is alkenoxy (Example 1) and alkoxy (Examples 2, 5, 6 and 7).

Similarly, pyrylium salts containing phenyl substituents bearing hydroxyl electron donor groups may be prepared using the corresponding hydroxy-substituted acetophenone and benzaldehyde derivatives.

Pyrylium salts containing phenyl substituents bearing acetamide electron donor groups may be prepared by treating mixtures of corresponding amine-substituted aromatic 1,3-dicarbonyl compounds and acetophenone derivatives with acetic anhydride by methods analogous to those outlined in "Comprehensive Organic Chemistry" by Barton and Ollis, Vol. 4, page 617 (Pergamon Press 1979). In this case, the anhydride functions as both a dehydrating/cyclisation and an acetylation reagent.

Pyrylium salts containing phenyl substituents bearing a phenoxy electron donor group can be prepared by the base catalysed addition of the corresponding phenoxy-substituted acetophenone to a substituted or unsubstituted 1,3-diphenylprop-1-en-3-one to yield the corresponding 1,5 diketone intermediate.
Cyclization and oxidation of the intermediate in the presence of acetic anhydride and ferric chloride yields the required pyrylium salt.
Thiohydrocarbyl and thioaryl groups may be introduced by reacting the corresponding thiohydrocarbyl and thioaryl substituted acetophenone and/or benzaldehyde by the method of Tsao referred to above.

Pyrylium salts containing phenyl groups bearing electron donor ester groups may be synthesised from the corresponding hydroxy phenyl (i.e. phenol) pyrylium salts by treatment with the required acid chloride. In the special case of phenylacetate-substituted pyrylium salts, this may be achieved by the treatment of mixtures of hydroxyphenyl-substituted 1,3-dicarbonyl compounds and acetophenone derivatives with acetic anhydride. In this case, the anhydride functions as both a dehydrating/cyclization and acetylation reagent.

All of the above reagents or starting compounds are either commercially available or cam be synthesised by well known procedures.

Preferably the pyrylium salt is a triarylpyrylium salt in which from one to three of the phenyl groups has an alkoxy or alkenoxy substituent. In a preferred embodiment the pyrylium salt has alkoxy or alkenoxy substitutents on the aryl groups in the 2,6-positions on the pyrylium ring. The alkoxy or alkenoxy substituents preferably are in the para- position on the phenyl ring(s).

Each phenyl ring may have one or more other substituents, for example alkyl substituents, provided that the substituent does not have electron withdrawing properties such as nitro or cyano groups, or groups known to inhibit cationic polymerisation, such as basic amino functions.

The hydrocarbyl group in the hydrocarbyloxy substituent on the or each aryl group preferably has from 1 to 20 carbon atoms, more particularly 1 to 5 carbon atoms; it may be saturated or unsaturated, straight chain or branched chain, and it may be functionalised or substituted.

Preferred pyrylium salts are of the formula

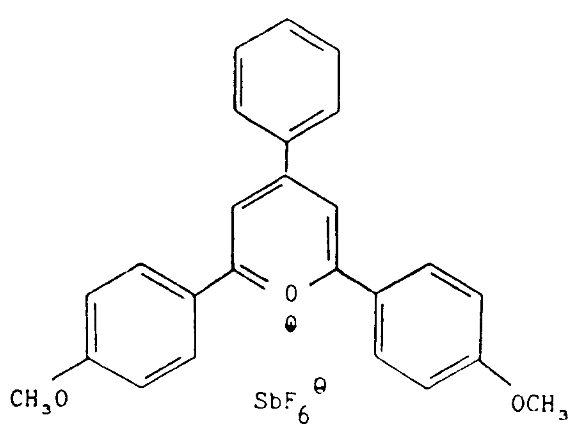

or

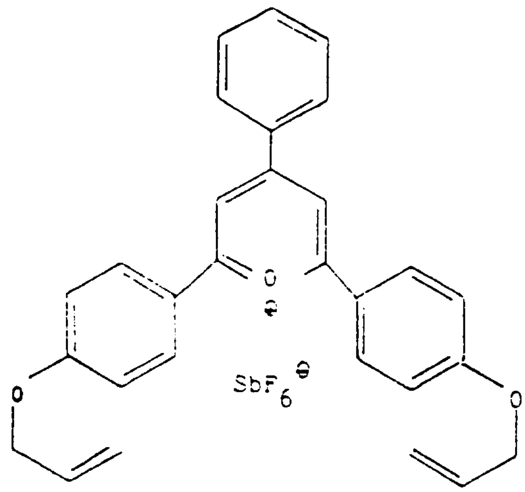

Particularly preferred materials are epoxides containing from 2 to 20 carbon atoms as well as epoxy resins.

The compositions of the present invention may be polymerised by exposure to ultraviolet radiation or visible light. They are useful in the production of surface coatings, adhesives, printing plates, printed circuits and generally in end uses where a one-package composition can be applied and cured by radiation.

It is surprising that the substitution of one or more electron donor groups into a triphenylpyrylium salt improves the stability of the photo-polymerisable composition without affecting its sensitivity to radiation.

Although the dark thermal stability of such compositions can be improved by addition of commercially available additives, it has been found that such additives, if included in quantities which are effective for stabilising purposes, have an adverse effect on the composition's response to irradiation.

The invention is illustrated in the following Examples:

Example 1:

The synthesis of 2,6-bis (4'-allyloxyphenyl)4-phenyl pyrylium hexafluoroantimonate.

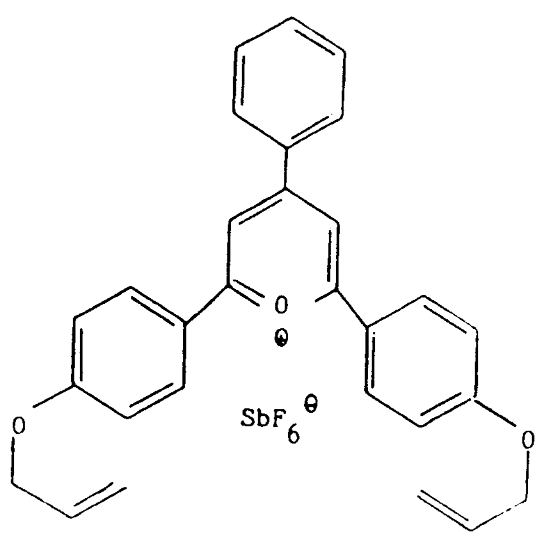

A solution of benzaldehyde (3.4g ; 0.032 moles), 4-allyloxyacetophenone (11.27 g ; 0.064 moles) and phosphorous oxychloride (18.4 g; 0.12 moles) was heated at 50 °C for 2 hours. The excess phosphorous oxychloride was removed under reduced pressure to yield a black residue. Alcohol (ca. 150mls, I.M.S.) was added to the residue and the boiling mixture was hot filtered to remove insolubles. Sodium hexaflouroantimonate (8.29g ; 0.032 moles) was added to the warm filtrate and the solution was allowed to stand in the fridge overnight. The red solid which had formed was filtered, washed with cold ethanol and then with cold ether and vacuum dried to give 6.03g (29% yield) of a red solid (Ref. no. 592/44).

An analytically pure sample was obtained by a column chromatographic separation (silica gel, acetone eluent) of a 0.5g portion of the material identified under Ref. No. 592/44. The elemental analysis obtained was as follows:

| | theory | found A | found B |
|---|---|---|---|
| %C | 52.97 | 52.39 | 52.02 |
| %H | 3.81 | 3.91 | 3.88 |
| % Halogen | 17.35 | 16.10 | --- |
| % Antimony | 18.57 | 18.62 | --- |

The close similarity between the theoretical and experimental values shows that the required compound has been formed.

Example 2:

The synthesis of 2,6-di (4'-methoxphenyl)-4-phenyl pyrylium hexafluoroantimonate.

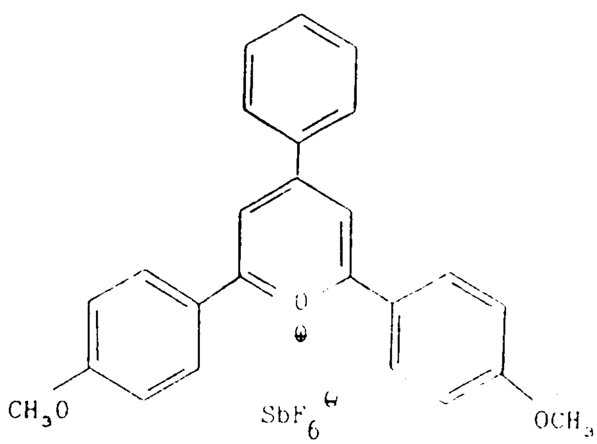

The above methoxyphenyl substituted pyrylium salt was prepared in a manner analogous to that described in Example 1. The red solid collected after filtration was recrystallised from art acetone/petrol solution and yielded 3.41 grains of the required salt in an analytically pure form. The elemental analyses obtained were as follows:

| | theory | found A | found B |
|---|---|---|---|
| % carbon | 49.59 | 48.52 | 48.12 |
| % hydrogen | 3.47 | 3.24 | 3.43 |
| % halogen | 18.84 | 19.94 | --- |
| % antimony | 20.17 | 18.15 | --- |

The similarity between the theoretical and experimental values indicates that the required compound was obtained. A UV/visible light spectrum was taken in methanol solution and 4 absorption maxima were observed as follows:

λmax 264nm (c = 21,300) ; λmax 322nm (c = 15,500) ; λmax 376nm (ε = 11,000) ; λmax 466nm (ε = 12,300).

Comparative Example A:

The synthesis of 2,4,6-triphenylpyrylium hexafluoroantimonate

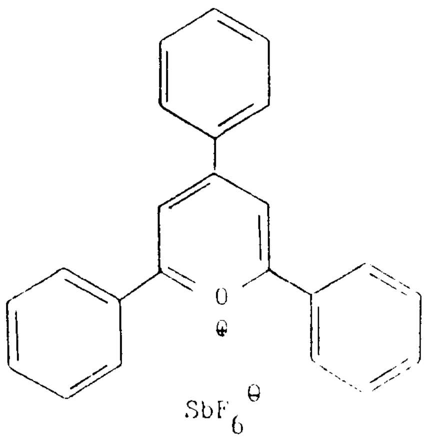

The same procedure was used to prepare the unsubstituted triphenyl pyrylium salt which was obtained as a yellow crystalline solid m.p. 285-290 ° C.

Example 3:

An epoxide composition was prepared which comprised equal parts by weight of EPON 828 (a bis phenol-A/glycol ether resin commercially available from Shell Chemicals Ltd.) and ERL-4221 (3,4-epoxycyclohexylmethyl-3,4-epoxycyclohexane carboxylate, commercially available from Union Carbide Corporation).

Photocurable formulations were prepared by dissolving 1% of the salts described in examples 1 and 2 and the comparative Example in the above epoxy mixture. In all cases the compositions were found to have cured to a hard, solvent-insoluble, material following 60 sec. exposure under a Lumatec UV mercury lamp.

The dark thermal stability of these materials were monitored. After 1 week the compositions containing the alkoxyphenyl substituted triphenylpyrylium salts (Examples 1 and 2) were unchanged, whereas the compositions containing the unsubstituted triphenylpyrylium salt (Comparative Example A) had become very viscous and showed signs of gellation. It was not useful as a liquid curable composition.

After 4 weeks compositions of Examples 1 and 2 showed no change in viscosity and were curable. After this time the composition of the Comparative Example A had gelled to a hard, insoluble material.

This result demonstrates that useful photocuring epoxide compositions can be prepared using alkoxy phenylsubstituted pyrylium salts.

EPON 828 and ERL-4221 are Trade Marks.

Example 4:

An epoxide composition was prepared by dissolving 1% of the pyrylium salt described in Example 2 in the monomer/resin blend described in Example 3.

Thin coatings of the composition were prepared on glass slides and exposed for 60 secs. under a Technocure LC 800 medium pressure mercury lamp with and without a polycarbonate UV filter. In both cases cured films were obtained which demonstrates that either UV radiation or visible light may be used to cure these compositions.

365mm Light intensity measurements were made at the curing surface and found to be as follows:

| | |
|---|---|
| No filter | 80mW/$cm^2$ |
| Polycarbonate filter | 1.5mW/$cm^2$ |

which demonstrates that the polycarbonate filter had effectively removed all of the UV radiation.

Comparative Example B:

It has been found possible to improve the dark, thermal stability of compositions containing the unsubstituted triphenylpyrylium salt (Comparative Example A), but in all cases this also resulted in a significant reduction or elimination of UV response.

The following composition was prepared,

| | % by weight |
|---|---|
| ERL-4221 | 96.75 |
| Propylene carbonate | 3.00 |
| 2,4,6-Triphenylpyryliumhexafluoroantimonate | 0.25 |

and stabilized with 2 different levels of bis (2,2,6,6-tetra-methyl-4-piperidyl) sebacate (TINUVIN 770 commercially available from Ciba Geigy). The thermal stability (at room temp. and at 82°C) and UV response were determined and are detailed in the following table:

| | No Stabilizer | 0.02% Tinuvin 770 | 0.1% Tinuvin 770 |
|---|---|---|---|
| Room Temperature Stability (Gellation Time) | 2 days | 6 days | 7 days |
| Stability 82 degrees C (Gellation Time) | 40 mins | 2 hrs. | 24 hrs. |
| % Gel Fraction on UV Exposure *(cf details below) | 100 | 91 | 0 |

*% Gel fraction was determined by accurately weighing the composition into a cylindrical plastic mould (8mm in diameter and 2mm in depth) such that the mould was filled completely. The mould containing the composition was then exposed for 40 secs. under a Technocure LC 800 UV lamp at a light intensity of 80 mW/cm$^2$ (365mm). The exposed sample was then removed from the mould and shaken with excess acetone (30 secs., mechanical shaker) to remove uncured material. After drying, the cured slug was re-weighed and the % gel fraction determined gravimetrically.

From the table it can be seen that at the low level of stabilizer there is a small, but not significant, improvement in thermal stability. There also is a small reduction in the UV response.

For the composition containing the high level of stabilizer a significant improvement in thermal stability is obtained, but there is no UV activity.

Example 5:

The synthesis of 2,6-di (4'-methoxyphenyl)-4-phenyl pyrylium hexafluoroarsenate.

The above methoxyphenyl substituted pyrylium salt was prepared in a manner analogous to that described in Example 1 but using sodium hexafluoroarsenate instead of sodium hexafluoro antimonate. A purified sample corresponding to a yield of 7.4% gave the following elemental analysis:

| | theory | found |
|---|---|---|
| % C | 53.78 | 52.91 |
| % H | 3.79 | 3.83 |
| % Halogen | 20.42 | 19.89 |
| % Arsenic | 13.41 | 14.5 |

The close similarity between the theoretical and experimental values shows that the required compound has been formed.

Example 6:

The synthesis of 2,6-diphenyl-4-(4'-methoxyphenyl) pyrylium hexafluoroantimonate.

The above methoxyphenyl substituted pyrylium salt was prepared in a manner analogous to that described in Example 1. A purified sample corresponding to a yield of 43% gave the following elemental analysis:

| | theory | found |
|---|---|---|
| % C | 50.10 | 49.47 |
| % H | 3.30 | 3.21 |

Example 7:

The synthesis of 2,4,6- tri(4'-methoxyphenyl) pyrylium hexafluoroantimonate.

The above methoxyphenyl substituted pyrylium salt was prepared in a manner analogous to that described in Example 1. A purified sample corresponding to a yield of 12% gave the following elemental analysis:

| | theory | found |
|---|---|---|
| % C | 49.13 | 49.25 |
| % H | 3.62 | 3.66 |

Comparative Example C:

Synthesis of 2,6-di (4'-methoxyphenyl)-4-phenyl pyrylium hexafluorophosphate.

The above methoxyphenyl substituted pyrylium salt was prepared in a manner analogous to that described in Example 1. A purified sample corresponding to a yield of 25% gave the following elemental analysis:

| | theory | found |
|---|---|---|
| % C | 58.37 | 57.34 |
| % H | 4.09 | 4.19 |

Example 8:

Epoxide compositions were prepared by dissolving 0.25% of the pyrylium salts shown in the table below in a mixture of 3% propylene carbonate and 96.75% ERL-4221. Thermal stability tests were conducted as described in Comparative Example B and UV Response was determined by measuring the UV exposure time

| Salt (Example No.) | Counterion | No. of MeO groups | Stability at 82 C (Gellation Time) | UV Response (Glass slide fixture time) |
|---|---|---|---|---|
| 2 | $SbF_6^-$ | 2 | 4 hrs. | 30 secs. |
| 6 | $SbF_6^-$ | 1 | 2 hrs. | 40 secs. |
| 7 | $SbF_6^-$ | 3 | 11 hrs. | 90 secs. |
| 5 | $AsF_6^-$ | 2 | 9 hrs. | 60 secs. |
| C | $PF_6^-$ | 2 | 12 hrs. | Does not fixture |

required to fixture 1mm thick microscope glass glides when 1 drop of the composition was placed between two slides. The slides were placed 15cms directly under the UV lamp of a Technocure LC800 light source operating at 200W/in (80 mW/cm$^2$). The irradiated slides were allowed to stand at room temperature for 5 mins before testing for fixture. The results obtained are listed in the above table.

These results demonstrate that both the nature of the counteranion and the number of methoxyphenyl substituents on the pyrylium salt affect the stability and UV Response of the expoxide composition : salts containing $SbF_6^-$ or $AsF_6^-$ anion are active whereas a salt containing $PF_6^-$ is inactive; increasing the number of alkoxyphenyl substituents increases the thermal stability of the composition although the UV response is reduced when the number of methoxy groups equals 3.

## Claims

**1.** A photopolymerisable composition comprising
(a) at least one epoxide compound or epoxy resin, and
(b) a pyrylium salt having at least one phenyl substituent bearing an electron donor group in the para- and/or ortho-position on the phenyl ring, and a hexafluoroantimonate or hexafluoroarsenate counterion.

**2.** A composition according to claim 1 wherein the electron donor group is selected from:
hydrocarbyloxy (-OR)
hydroxy (-OH)
amide ($-NHCOCH_3$)
phenoxy (-O Ar)
thiohydrocarbyl (-SR)
thioaryl (-SAr)
phenyl ester (-OCOR)
wherein R represents hydrocarbyl and Ar represents aryl.

**3.** A photopolymerisable composition according to claim 1 or 2 wherein the electron donor group is an alkoxy or alkenoxy group.

**4.** A composition according to claim 3 wherein the pyrylium salt is a triarylpyrylium salt in which from one to three of the phenyl groups has an alkoxy or alkenoxy substituent.

**5.** A composition according to claim 4 wherein the pyrylium salt has alkoxy or alkenoxy substitutents on the phenyl groups in the 2,6-positions on the pyrylium ring.

**6.** A composition according to any of claims 3-5 wherein the alkoxy or alkenoxy substituent(s) are in the para- position.

**7.** A composition according to any of claims 1-6 wherein the pyrylium salt is 2,6-bis (4'allyloxyphenyl)-4-phenyl pyrylium hexafluoroantimonate.

**8.** A composition according to any of claims 1-6 wherein the pyrylium salt is 2,6-di (4'-methoxyphenyl)-4-phenyl pyrylium hexafluoroantimonate.

**9.** A composition according to any of claims 1-6 wherein the pyrylium salt is 2,6-di (4'-methoxyphenyl)-4-phenyl pyrylium hexafluoroarsenate.

10. A composition according to any of claims 1-6 wherein the pyrylium salt is 2,6-diphenyl-4-(4'methoxyphenyl) pyrylium hexafluoroantimonate.

11. A composition according to any of claims 1-6 wherein the pyrylium salt is 2,4,6- tri(4'methoxyphenyl) pyrylium hexafluoroantimonate.

12. A composition according to any of claims 1-11 herein component (a) is an epoxy resin.

13. A composition according to any of claims 1 to 11 wherein component (a) is an epoxide.

14. A composition according to claim 13 wherein the epoxide is 3,4-epoxycyclohexylmethyl-3,4-epoxycyclohexanecarboxylate.

**Patentansprüche**

1. Photopolymerisierbare Zusammensetzung umfassend
   (a) mindestens eine Epoxidverbindung oder ein Epoxyharz und
   (b) ein Pyryliumsalz mit mindestens einem Phenylsubstituenten, der eine Elektronendonorgruppe in der para- und/oder ortho-Position am Phenylring trägt, und einem Hexafluorantimonat- oder Hexafluorarsenat-Gegenion.

2. Zusammensetzung nach Anspruch 1, worin die Elektronendonorgruppe ausgewählt ist aus:
   Hydrocarbyloxy (-OR)
   Hydroxy (-OH)
   Amid ($-NHCOCH_3$)
   Phenoxy (-OAr)
   Thiohydrocarbyl (-SR)
   Thioaryl (-SAr)
   Phenylester (-OCOR),
   worin R Hydrocarbyl und Ar Aryl darstellt.

3. Photopolymerisierbare Zusammensetzung nach Anspruch 1 oder 2, worin die Elektronendonorgruppe eine Alkoxy- oder Alkenoxygruppe ist.

4. Zusammensetzung nach Anspruch 3, worin das Pyryliumsalz ein Triarylpyryliumsalz ist, worin eine bis drei der Phenylgruppen einen Alkoxy- oder Alkenoxysubstituenten hat.

5. Zusammensetzung nach Anspruch 4, worin das Pyryliumsalz Alkoxy- oder Alkenoxysubstituenten an den Phenylgruppen in den 2,6-Positionen am Pyryliumring hat.

6. Zusammensetzung nach einem der Ansprüche 3 - 5, worin der (die) Alkoxy- oder Alkenoxysubstituent(en) sich in para-Position befindet(en).

7. Zusammensetzung nach einem der Ansprüche 1 - 6, worin das Pyryliumsalz 2,6-Bis(4'-allyloxypheny)-4-phenylpyryliumhexylfluorantimonat ist.

8. Zusammensetzung nach einem der Ansprüche 1-6, worin das Pyryliumsalz 2,6-Di(4'-methoxyphenyl)-4-phenylpyryliumhexylfluorantimonat ist.

9. Zusammensetzung nach einem der Ansprüche 1-6, worin das Pyryliumsalz 2,6-Di(4'-methoxyphenyl)-4-phenylpyryliumhexylfluorarsenat ist.

10. Zusammensetzung nach einem der Ansprüche 1-6, worin das Pyryliumsalz 2,6-Diphenyl-4-(4'-methoxyphenyl)pyryliumhexylfluorantimonat ist.

11. Zusammensetzung nach einem der Ansprüche 1-6, worin das Pyryliumsalz 2,4,6-Tri(4'-methoxyphenyl)-pyryliumhexylfluorantimonat ist.

**12.** Zusammensetzung nach einem der Ansprüche 1 - 11, worin Komponente (a) ein Epoxyharz ist.

**13.** Zusammensetzung nach einem der Ansprüche 1 bis 11, worin Komponente (a) ein Epoxid ist.

**14.** Zusammensetzung nach Anspruch 13, worin das Epoxid 3,4-Epoxycyclohexylmethyl-3,4-epoxycyclohexancarboxylat ist.

**Revendications**

**1.** Une composition photopolymérisable comprenant
(a) au moins un époxyde ou une résine époxy, et
(b) un sel de pyrylium ayant au moins un substituant phényle portant un groupe donneur d'électrons en position *para* et/ou ortho sur le noyau phénylique, et un ion complémentaire hexafluorantimoniate ou hexafluorarséniate.

**2.** Une composition selon la revendication 1, dans laquelle le groupe donneur d'électrons est choisi parmi :
hydrocarbyloxy (-OR)
hydroxyle (-OH)
amide ($-NHCOCH_3$)
phénoxy (-OAr)
thiohydrocarbyle (-SR)
thioaryle (-SAr)
ester de phényle (-OCOR)
où R représente un groupe hydrocarbyle et Ar représente un groupe aryle.

**3.** Une composition photopolymérisable selon la revendication 1 ou 2, dans laquelle le groupe donneur d'électrons est un groupe alcoxy ou alcénoxy.

**4.** Une composition selon la revendication 3, dans laquelle le sel de pyrylium est un sel de triarylpyrylium dans lequel un à trois des groupes phényle portent un substituant alcoxy ou alcénoxy.

**5.** Une composition selon la revendication 4, dans laquelle le sel de pyrylium comporte des substituants alcoxy ou alcénoxy sur les groupes phényle aux positions 2,6 du noyau de pyrylium.

**6.** Une composition selon l'une quelconque des revendications 3 à 5, dans laquelle le ou les substituants alcoxy ou alcénoxy sont en position *para*.

**7.** Une composition selon l'une quelconque des revendications 1 à 6, dans laquelle le sel de pyrylium est l'hexafluorantimoniate de 2,6-bis(4'-allyloxyphényl)-4-phénylpyrylium.

**8.** Une composition selon l'une quelconque des revendications 1 à 6, dans laquelle le sel de pyrylium est l'hexafluorantimoniate de 2,6-di(4'-méthoxyphényl)-4-phénylpyrylium.

**9.** Une composition selon l'une quelconque des revendications 1 à 6, dans laquelle le sel de pyrylium est l'hexafluorarséniate de 2,6-di(4'-méthoxyphényl)-4-phénylpyrylium.

**10.** Une composition selon l'une quelconque des revendications 1 à 6, dans laquelle le sel de pyrylium est l'hexafluorantimoniate de 2,6-diphényl-4-(4'-méthoxyphényl) pyrylium.

**11.** Une composition selon l'une quelconque des revendications 1 à 6, dans laquelle le sel de pyrylium est l'hexafluorantimoniate de 2,4,6-tri(4'-méthoxyphényl)pyrylium.

**12.** Une composition selon l'une quelconque des revendications 1 à 11, dans laquelle le composant (a) est une résine époxy.

**13.** Une composition selon l'une quelconque des revendications 1 à 11, dans laquelle le composant (a) est un époxyde.

**14.** Une composition selon la revendication 13, dans laquelle l'époxyde est le 3,4-époxycyclohexane-carboxylate de 3,4-époxycyclohexylméthyle.